# EUROPEAN PATENT APPLICATION

(11) **EP 3 275 484 A1**
(43) Date of publication of application: **31.01.2018**
(21) Application number: 16181180.7
(22) Date of filing: 26.07.2016
(51) Int. Cl.: A61M 5/32, A61M 25/06

(54) **PROTECTIVE ELEMENT FOR MEDICAL NEEDLE ASSEMBLIES**

(71) Applicant: Delta Med S.p.A., 46019 Viadana (IT)
(72) Inventor: Balboni, Alessandro, 46019 Viadana (MN) (IT)
(74) Representative: Wegner, Hans

(57) **Abstract**

The present invention concerns a protective element (1; 2; 3) for a medical needle assembly, the medical needle assembly comprising a housing (4) and a needle (5) having a needle shaft and a needle tip. The protective element (1; 2; 3) comprises a proximal wall (10; 20; 30) which is transversely arrangeable on the needle shaft, the proximal wall (10; 20; 30) comprising a first opening (11; 21; 31) through which the needle shaft can extend, a protective arm (12; 22; 32) extending from the proximal wall (10; 20; 30) in a distal direction, the protective arm (12; 22; 32) being adapted for covering the needle tip in an activated position, and two proximal side walls (13; 23; 33) extending from opposing sides of the proximal wall (10; 20; 30) in the distal direction, the two proximal side walls (13; 23; 33) being adapted for engaging with a cavity (6) of the housing (4) in a ready position.

## Description

### 1. Technical field

The present invention generally relates to the field of medical needle assemblies, and more particularly to protective elements for the same which prevent inadvertent needle pricks.

### 2. The prior art

Various safety apparatuses for medical needle assemblies including e.g. hypodermic needles or intravenous catheters are known in the art. These safety apparatuses typically comprise a protective element, such as a spring clip, for covering the needle tip after use.

For example, WO 2014/097110 A1 of applicant discloses a cannula-needle with a protective member for protecting the needle tip which is movable from a disabled position to an enabled position. WO 99/08742 discloses a resilient needle guard which is configured to snap into a position in which it is clamped onto the needle shaft and in which its distal wall blocks access to the needle tip. EP 1 344 544 A1 discloses a protector displaceable on the needle shaft which covers the needle tip.

However, the known protective elements have a number of drawbacks. Some of them require an additional housing for housing the protective element, which results in an assembly which is complex to manufacture and difficult to handle. Further, the known protective elements tend to twist on the needle shaft in an uncontrolled manner when activated, thereby creating the risk of the needle escaping from the protective element after the needle has been withdrawn.

It is therefore the technical problem underlying the present invention to provide a protective element for a medical needle assembly which safely and reliably covers the needle tip, is easy to use and/or easy to manufacture and thereby at least partly overcomes the above explained disadvantages of the prior art.

### 3. Summary of the invention

The invention is defined by the independent claims. Advantageous modifications of embodiments of the invention are defined in the dependent claims.

In the embodiment of claim 1, the protective element comprises a proximal wall, a protective arm and two proximal side walls. The proximal wall is transversely arrangeable on the needle shaft of the needle assembly (i.e. preferably essentially perpendicular to the needle shaft) and comprises a first opening through which the needle shaft can extend. The protective arm extends from the proximal wall in a distal direction and is adapted for covering the needle tip in an activated position. The two proximal side walls extend from opposing sides of the proximal wall in the distal direction.

According to the invention, the two proximal side walls are arranged, shaped and/or oriented such that they are adapted for engaging with a cavity of the housing in a ready position. This way, the orientation of the proximal side walls is fixed relative to the housing and therefore also relative to the needle axis. Due to the connection between the proximal side walls and the proximal wall, also the proximal wall's orientation is fixed relative to the needle axis. This advantageously provides stability to the protective element and prevents an uncontrolled tilting of the protective element as it snaps from the ready position into the activated position, as will be explained further below.

In one aspect of the invention, the protective arm may comprise a distal wall comprising a second opening through which the needle shaft can extend in the ready position. Accordingly, the distal wall attached to the protective arm allows the needle to pass through the second opening when the needle is retracted and forms a barrier for the needle tip once it is retracted inside the protective element when the same snaps into the activated position.

The protective element may further comprise an essentially stationary arm extending from the proximal wall in the distal direction, preferably opposite to the protective arm, the essentially stationary arm terminating in a curved lip. The essentially stationary arm provides further stability to the protective element, since the curved lip can slide on the needle shaft when the needle is retracted, thereby keeping the orientation of the protective element relative to the needle shaft.

Alternatively, the essentially stationary arm may comprise a transverse segment comprising a third opening through which the needle shaft can extend. This way, the stability is further improved, since the needle cannot escape the protective element laterally due to the transverse segment through which the needle passes.

Preferably, the protective arm is flexible relative to the proximal wall, i.e. it is preferably bent to the outside in the ready position and snaps into the activated position in which it covers the needle tip while the proximal wall keeps its orientation relative to the needle axis.

As shown in the figures which will be explained further below, the protective element is in the above-described embodiments preferably essentially G-shaped.

In an alternative embodiment, the protective arm may comprise a transverse segment comprising a third opening through which the needle shaft can extend. This way, the stability is even more improved due to three guiding structures provided for the needle shaft to pass through.

The portion of the protective element proximal from and including the transverse segment may be essentially stationary and the portion of the protective element distal from the transverse segment may be flexible relative to the transverse segment. The protective element is thereby particularly stable, since its proximal portion provides a large static supporting base for slidably arranging the protective element on the needle shaft.

The protective element may also comprise an essentially stationary arm extending from the proximal wall in the distal direction towards the transverse segment such that the transverse segment is essentially stationary relative to the proximal wall. In other words, the stationary arm provides a barrier for the movement of the transverse segment, such that the proximal portion of the protective element remains static while only its distal portion can flex between the ready position and the activated position.

The protective element may in this manner be essentially S-shaped, as will be further explained in the detailed description below.

Preferably, the first, second and / or third openings do not contact the needle shaft. This may be achieved by way of the engagement of the protective element inside the cavity of the housing and the geometry of the protective element. Advantageously, when the needle shaft does not contact the opening(s) during retraction, this results in a particularly smooth operation.

In addition or alternatively, the protective arm may comprise a distal end segment adapted for engaging with the cavity of the housing in the ready position, thereby forcing the protective arm into a first position in which the first opening and the second opening are aligned relative to the needle axis, so that the needle shaft can pass through the openings. When removed from the housing, the protective arm may be adapted for moving into a second position in which the second opening is misaligned relative to the needle axis. Accordingly, the needle tip is safely held within the protective element and can no longer escape from the second opening.

The protective element may further comprise two distal side walls forming, together with the distal wall, a box-shaped distal portion of the protective element adapted for collecting blood leaking from the needle tip in the activated position.

The present invention is also directed to a medical needle assembly comprising a needle having a needle shaft and a needle tip, a protective element according to any of the above-described embodiments, and a housing, preferably a cannula holder, having a cavity adapted for receiving the protective element.

### 4. Short description of the drawings

In the following detailed description, presently preferred embodiments of the invention are further described with reference to the following figures:
- Figs. 1a-f:: A protective element according to a first embodiment of the invention;
- Figs. 2a-f:: A protective element according to a second embodiment of the invention;
- Figs. 3a-f:: A protective element according to a third embodiment of the invention;
- Figs. 4a-b:: Detail views of the distal end of a needle illustrating the engagement between the needle and the protective element according to embodiments of the invention;
- Figs. 5a-c:: Detail views of a cannula holder and the protective element according to embodiments of the invention;
- Fig. 6a:: A needle assembly according to embodiments of the invention in the ready position;
- Fig. 6b:: The needle assembly of Fig. 6a in an intermediate position;
- Fig. 6c:: The needle assembly of Figs. 6a and 6b in the activated position;
- Fig. 7:: A protective element according to a modification of the third embodiment of the invention.

### 5. Detailed description of preferred embodiments

In the following, presently preferred embodiments of the invention are described with respect to a protective element for a medical needle assembly which serves for safely covering the sharp and potentially contaminated needle tip after it has been withdrawn from the patient. The medical needle assembly may be used for various applications, wherein exemplary applications may include without limitation hypodermic needles, intravenous catheters or arterial catheters.

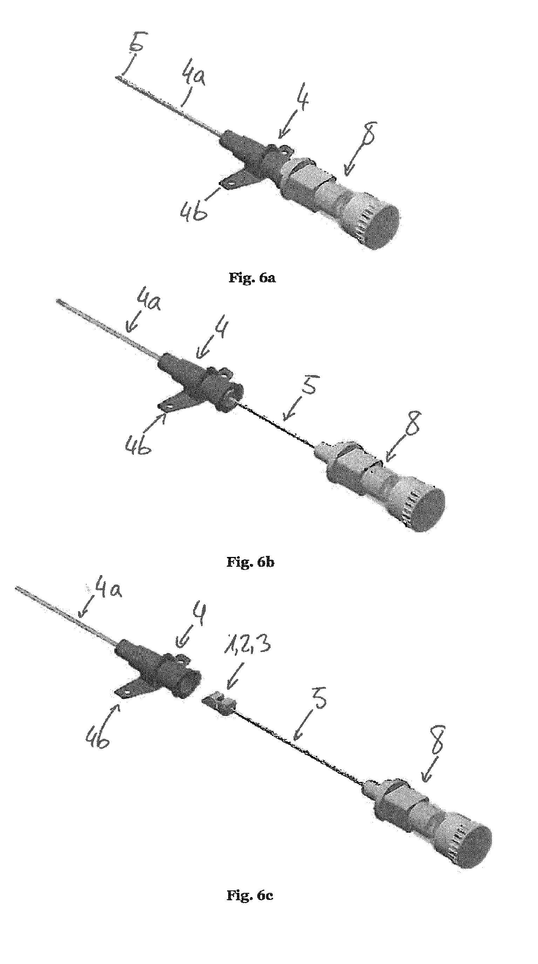

The protective elements 1, 2, 3 according to embodiments of the invention are generally configured to be used in connection with medical needle assemblies such as the hypodermic needle assembly exemplarily illustrated in Figs. 6a-c. Although a hypodermic needle assembly will be used as an example in the following description, it will be appreciated that the present invention is likewise applicable in all sorts of medical needle assemblies.

As can be seen in Fig. 6a, the needle assembly comprises a housing 4, preferably a cannula holder, wherein a flexible, generally tube-shaped cannula 4a is provided at the distal end of the housing 4 (the term "distal" as used herein indicates the direction towards the needle tip). In the illustrated embodiment, the cannula holder 4 comprises two lateral wings 4b which serve for fixing the cannula holder 4 to the patient's skin during operation. However, the wings 4b are optional and the embodiments of the invention described herein are generally configured for use with any suitable cannula holder or housing 4. The needle assembly further comprises a needle holder 8 at the distal end of which a needle 5 is provided. The needle 5 comprises a needle shaft and a needle tip at its distal end, wherein the longitudinal direction of the needle shaft defines a needle axis. Fig. 6a shows the assembly in the so-called "ready position" in which the protective element 1, 2, 3 of the invention (not shown in Fig. 6a) is housed inside the housing 4 and is slidably provided on the needle shaft. In the ready position, the needle 5 extends through the housing 4 and the cannula 4a.

When the housing 4 for the protective element 1, 2, 3 is formed by the cannula holder, this results in a particularly simple needle assembly, since no separate housing for the protective element 1, 2, 3 is needed.

During operation, after the needle 5 has been inserted into the patient, the operator may hold the housing 4 in place with one hand or otherwise fix the housing 4 to the patient. With the other hand, the operator may grasp the needle holder 8 and pull the same in the proximal direction (the term "proximal" as used herein indicates the direction away from the needle tip), thereby withdrawing the needle 5 from the patient while the cannula 4a remains inserted in the patient. In this interim state, the protective element 1 still stays within the housing 4 as the needle shaft travels through the housing 4 and the protective element 1 (see Fig. 6b).

When the needle 5 is further withdrawn proximally, the needle tip eventually enters the protective element 1 which then snaps into the so-called "activated position" in which it safely guards the needle tip, as will be explained further below. In this position, the needle tip and the protective element 1 escape proximally from the housing 4 upon further proximal movement of the needle holder 8, as shown in Fig. 6c. To this end, the housing 4 preferably has an open proximal end, and more preferably a closed distal end (closed in the sense that only the cannula 4a extends from the distal end of the housing 4).

Although Figs. 6a-c illustrate the needle assembly in connection with one particular embodiment of the protective element 1, it shall be noted that the above explained needle assembly may be used with any of the inventive protective elements 1 which will be described in the following.

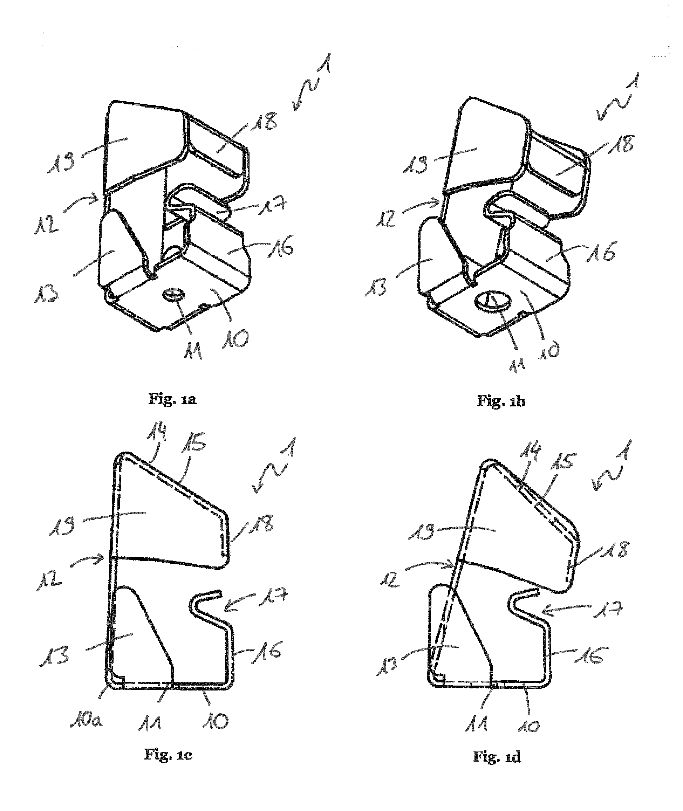

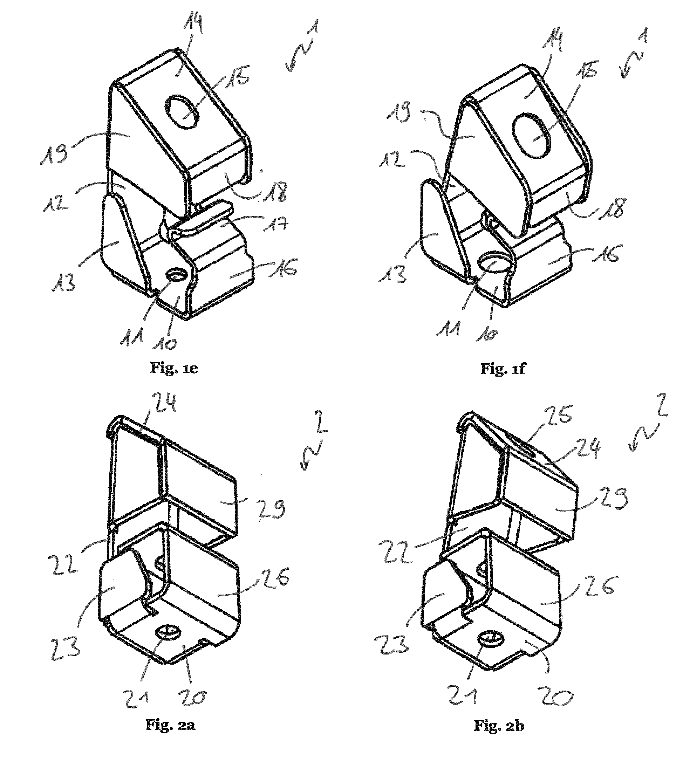

Figs. 1a-f illustrate a first embodiment of a protective element 1 of the invention. In Figs. 1a, 1c and 1e, the protective element 1 is shown in the ready position in which the protective element 1 is slidably provided on the needle shaft (not shown). Turning first to the side view in Fig. 1c, it can be seen that the protective element 1 is of an asymmetric shape with respect to the needle axis and/or is essentially G-shaped. The protective element 1 comprises a proximal wall 10 which is preferably oriented transversely, i.e. essentially perpendicular, to the needle axis. A first opening 11 (best seen in Fig. 1a) is provided in the proximal wall 10 and has a diameter which is larger than the diameter of the needle shaft so that the needle 5 can extend through the opening 11 and therefore the protective element 1 can slide on the needle shaft, as explained above.

A protective arm 12 may extend from the proximal wall 10 in a distal direction. The protective arm 12 is preferably flexible with respect to the proximal wall 10 in that it can pivot around the edge 10a (see Fig. 1c) between the proximal wall 10 and the protective arm 12.

At the distal end of the protective arm 12 a distal wall 14 may be provided which has a second opening 15 through which the needle shaft can pass. In the depicted embodiment, the distal wall 14 extends obliquely across the needle axis, i.e. it is not in parallel to the proximal wall 10, and the second opening 15 has an elliptical shape in order to provide a passageway for the needle (note that the elliptical opening 15 appears essentially as if it was circular when seen from the distal direction due to the oblique arrangement of the distal wall 14). The second opening 15 may, however, have any shape as long as the created passageway is large enough to allow the engaging means 7 to pass through, as will be explained further below. Generally, the smaller the opening, the better the protective element 1 can cover the needle tip.

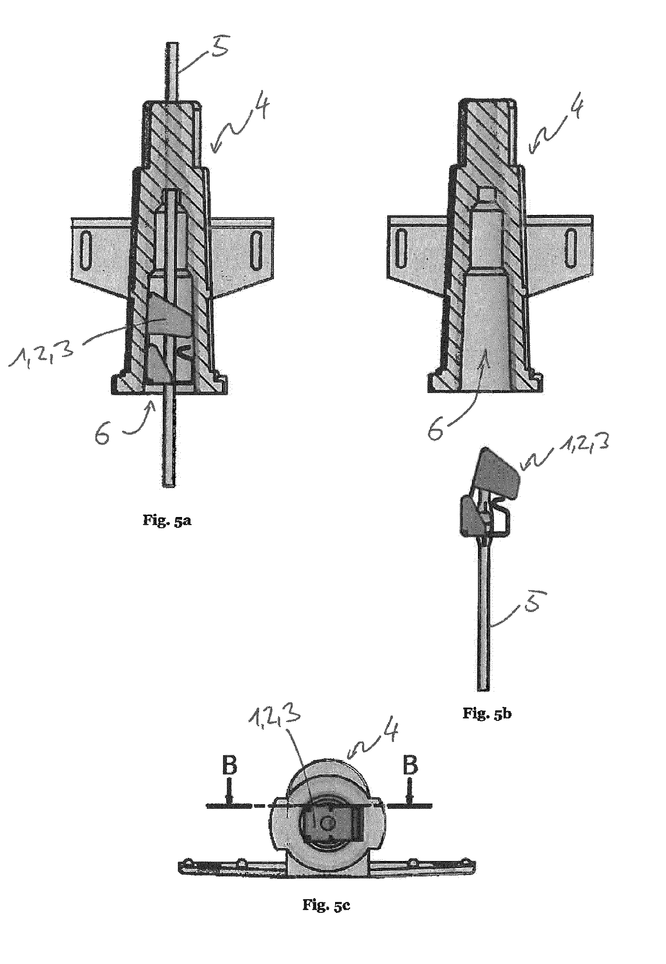

The distal wall 14 may migrate into a distal end segment 18. The distal portion of the protective element 1 formed by the distal wall 14 and the distal end segment 18 is preferably arranged such that when the protective element 1 is housed inside the cavity 6 of the housing 4, the protective arm 12 is urged laterally to the outside, i.e. away from the needle shaft, such that the first opening 11 and the second opening 15 come into alignment (see Fig. 1c) and thus the needle can pass through both openings 11 and 15 (see also Fig. 5a).

When the protective element 1 is removed from the housing 4, the force acting on the distal end segment 18 is removed and the protective arm 12 moves into its natural position, which is shown in Figs. 1b, 1d and 1f. As can be seen, the second opening 15 is now misaligned relative to the first opening 11 such that the sharp needle tip is safely covered by the distal portion of the protective element 1 (see also Fig. 5b). This second position is referred to as the "activated position".

In this manner, the protective element 1 can be used similar to what is disclosed in WO 2014/097110 of applicant, the contents of which are entirely incorporated by reference herein.

Similar to the protective element disclosed in WO 2014/097110, also the protective element 1 of the invention may comprise two distal side walls 19 near the distal end of the protective element 1. This way, the distal portion of the protective element 1 has a box-like shape with an open proximal side facing the proximal wall 10.

The two distal side walls 19 and/or the box-like shape of the distal portion of the protective element 1 have the effect of preventing blood from spilling out of the protective element 1 when the protective element 1 is removed from the housing 4**.** This is because the protective element 1 preferably has an overall size of only a few millimeters, so that the surface tension of blood on the inside of the distal side walls 19 is sufficient to keep the blood inside the protective element 1**.**

The protective element 1 preferably further comprises two proximal side walls 13 (only one of which is visible in Figs. 1a-f) extending from opposite sides of the proximal wall 10 in an essentially right angle, so that the proximal side walls 13 extend in the distal direction and are generally perpendicular to the protective arm 12.

In the ready position, the two proximal side walls 13 engage the inner surface of the cavity of the housing 4 so that the protective element 1 is initially held within the housing 4 by means of a friction fit when the needle is retracted.

According to the invention, the proximal side walls 13 serve a stabilizing function for the protective element 1**.** As explained above, the proximal wall 10 and the protective arm 12 are pivotable relative to each other (compare Figs. 1c and 1d). Therefore, when the protective element 1 snaps from the ready position (Fig. 1c) into the activated position (Fig. 1d) it could normally happen that the proximal wall 10 twists at least slightly out of its essentially right-angled orientation relative to the needle axis. Such an uncontrolled twisting is disadvantageous, since it is detrimental to the stability of the protective element 1 on the needle shaft and could in the worst case even clamp the protective element 1 to the needle shaft in a twisted manner, thereby avoiding a proper function and operation thereof.

Such an uncontrolled twisting of the proximal wall 10 and thus the entire protective element 1 is avoided by means of the proximal side walls 13. Since the side walls 13 are arranged such that their outer edges engage with the inner surface of the cavity 6 of the housing 4 (see Fig. 5a), the orientation of the proximal wall 10 is fixed relative to the needle axis as long as the protective element 1 is housed inside the housing 4**.** When the protective element 1 snaps into the activated position while it is still inside the housing 4, the proximal side walls 13 (due to their engagement with the cavity 6) avoid a twisting of the proximal wall 10, so that only the protective arm 12 pivots. Once the protective element 1 has been activated in this controlled manner, it is also clamped to the needle in a controlled manner and correct orientation and can be withdrawn from the housing 4 (see Fig. 5b).

In addition to the aforementioned stabilizing function, the two proximal side walls 13 also contribute to the effect of preventing blood from spilling out of the protective element 1 when the protective element 1 is removed from the housing 4, similar to the distal side walls 19 explained above.

Moreover, the protective element 1 may comprise an essentially stationary arm 16 extending distally from the proximal wall 10 and opposite to the protective arm 12. The arm 16 is stationary in the sense that its orientation with respect to the proximal wall 10 (preferably right-angled) is fixed, i.e. the stationary arm 16 cannot pivot relative to the proximal wall 10. In other words, the angle between the arm 16 and the proximal wall 10 is always the same in both the ready and the activated position, i.e. the edge between the proximal wall 10 and the arm 16 has no memory. On the other side, the angle between the proximal wall 10 and the protective arm 12 is flexible, as explained above. This can be achieved by providing an angle less than 90° between the proximal wall 10 and the protective arm 12 (preferably an angle of approx. 75°) when the protective element 1 is manufactured to gives this section of the protective element 1 resilience. Then, the protective arm 12 is bent to the outside (preferably to approx. 86°) when in the ready position. In this way, the protective arm 12 has the shape memory that makes it flip and activate the protective element 1.

The essentially stationary arm 16 of the first embodiment terminates in a curved lip 17 (see Fig. 1c and 1d). The curved lip 17, more precisely its curvature, can slide on the needle shaft during retraction of the needle, thereby stabilizing the protective element 1 and at the same time reducing the friction created by the sliding movement, as compared to the design disclosed in WO 2014/097110.

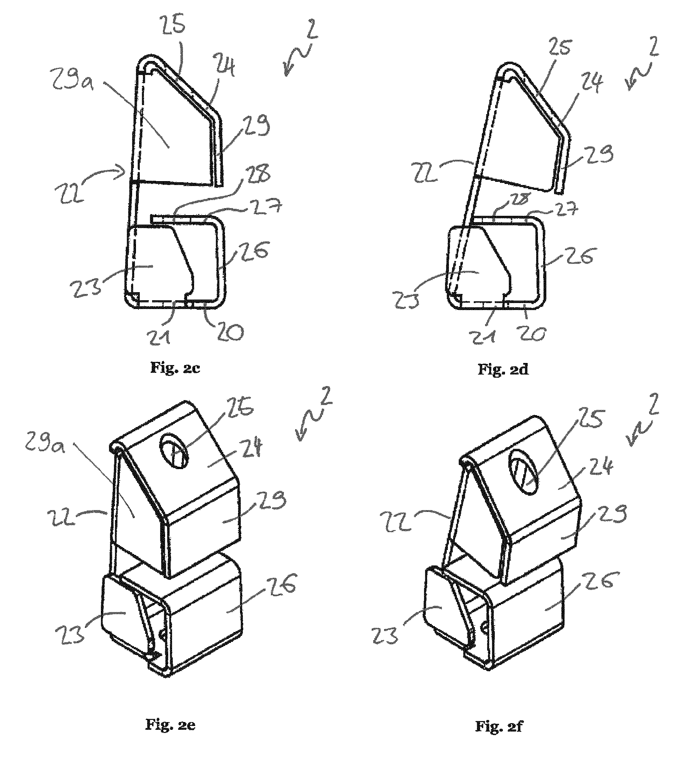

A second embodiment of the protective element 2 of the invention is illustrated in Figs. 2a-f. As can be seen, the protective element 2 also has a generally asymmetric shape with respect to the needle axis and/or is essentially G-shaped. It may comprise a proximal wall 20 with a first opening 21, a protective arm 22, a distal wall 24 with a second opening 25, a distal end segment 29, two distal side walls 29a and/or two proximal side walls 23. These elements are essentially identical to the corresponding elements of the first embodiment described above and thus the disclosure further above equally applies here.

Different from the first embodiment, the essentially stationary arm 26 terminates in a transverse segment 27 (transverse with respect to the needle axis; best seen in Figs. 2c and 2d). The transverse segment 27 comprises a third opening 28 through which the needle shaft can pass. This transverse segment 27 further improves the stability of the protective element 1 in that it prevents the needle from escaping laterally, or in other words, to avoid a lateral flipping of the protective element 1 relative to the needle in the activated position. The third opening 28 is preferably circular, however, any shape may be used. Also here, the smaller the opening 28 is, the better, since it can give a stricter constraint to the needle.

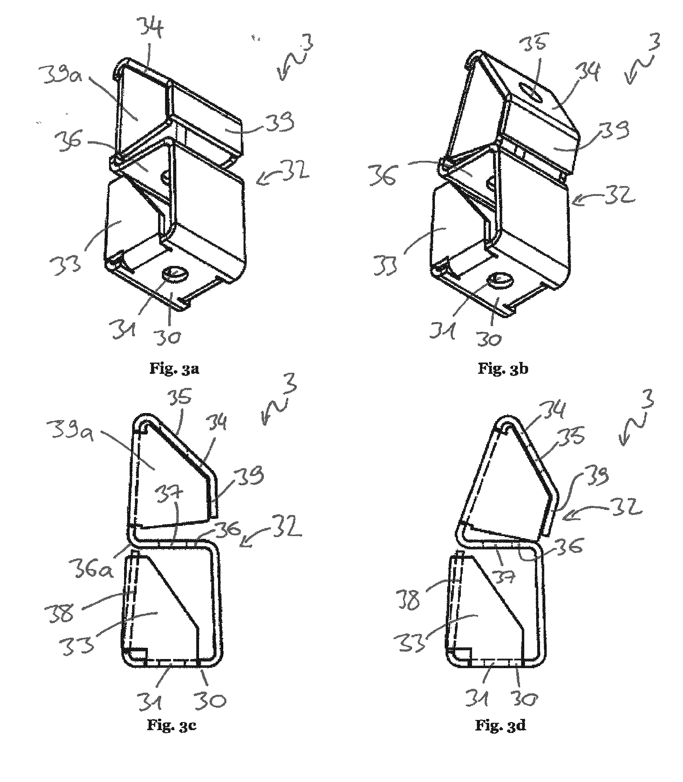

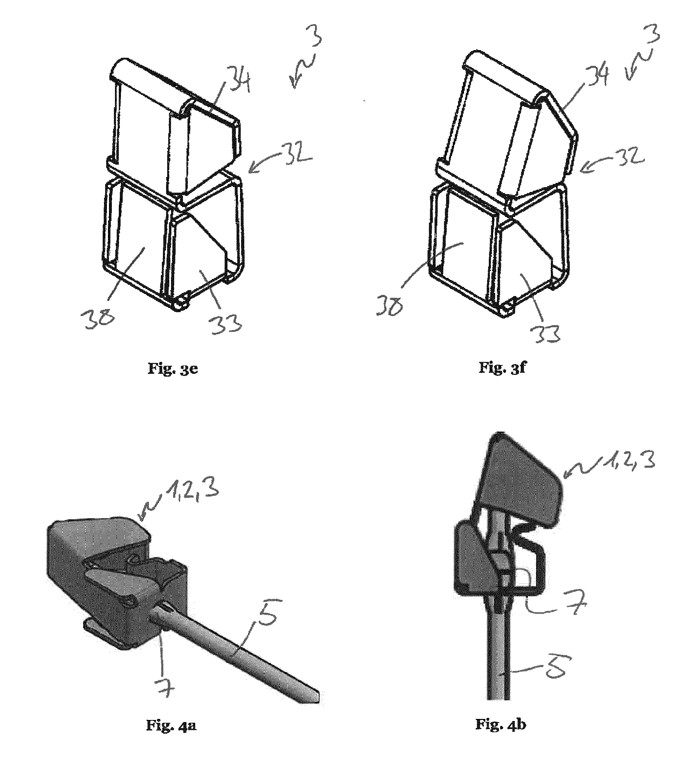

A third embodiment of the protective element 3 of the invention is illustrated in Figs. 3a-f. The protective element 3 may comprise a proximal wall 30 with a first opening 31, a protective arm 32, a distal wall 34 with a second opening 35, a distal end segment 39, two distal side walls 39a and/or two proximal side walls 33. These elements are essentially identical to the corresponding elements of the first and second embodiments described above and thus the disclosure further above equally applies here.

Also the protective element 3 of the third embodiment is generally asymmetric in shape with respect to the needle axis. Different from the first and second embodiment, the protective element 3 of the third embodiment is essentially S-shaped. Accordingly, the protective arm 32 extends from the proximal wall 30 in the distal direction and bends into the transverse segment 36 which is therefore arranged transversely with respect to the needle 5 and crosses the needle shaft. On the other side of the needle shaft, the transverse segment 36 bends into a segment which is again essentially parallel to the needle axis (or has a defined angle with respect to the needle axis), wherein this parallel segment then bends into the distal wall 34 (see Fig. 3c).

Importantly, the proximal portion (as seen in Fig. 3c) of the protective element 3 formed by the segments proximal to and including the transverse segment 36 is essentially stationary, i.e. these segments do not bend relative to each other during use of the protective element 3. The distal portion of the protective element 3 formed by the segments distal to the transverse segment 36 are flexible in that the distal portion can tilt between the ready position and the activated position by means of a hinging movement around the edge 36a. In addition, the essentially stationary arm 38 may serve as a support for the transverse segment 36 in that the distal end of the stationary arm 38 terminates just below the edge 36a of the transverse segment 36. This way, the transverse segment 36 cannot bend relative to the proximal wall 30 since it hits the distal end of the stationary arm 38. Accordingly, the stability of the protective element 1 is further improved in this embodiment.

In all embodiments, the first opening 11, 21, 31, the second opening 15, 25, 35 and/or the third opening 28, 37 preferably does not contact the needle shaft in the ready position, and most preferably none of the openings contacts the needle shaft in the ready position. In other words, the diameter of the respective opening is chosen such that the needle shaft can pass therethrough without touching the rim of the opening. This avoids friction between the needle shaft and the opening(s) during retraction of the needle and therefore achieves a particularly smooth operation of the assembly. Preferably, the only case of contact is when the engaging means 7 of the needle shaft engages the first opening 11,21,31, as will be explained in the following.

As illustrated in Figs. 4a-b, in all embodiments the needle 5 comprises an engaging means 7 near the needle tip for preventing the protective element 1, 2, 3 from being withdrawn from the needle tip. The engaging means 7 has a diameter greater than that of the needle shaft and greater than that of the first opening 11, 21, 31. In the embodiment of Figs. 4a-b, the engaging means 7 is provided in the form of a bulge, however, any other sort of increased diameter segment can be used e.g. by means of a needle crimp or the like, as known to the person skilled in the art.

Figs. 5a-c illustrate the interaction of the protective element 1, 2, 3 with the housing 4**.** Although only the first embodiment of protective element 1 is shown in Figs. 5a-c, it shall be understood that the concepts described herein equally apply to the other embodiments of the protective element 1, 2, 3 of the invention.

In the ready position shown in Fig. 5a, the protective element 1, 2, 3 is housed inside the cavity 6 of the housing 4. Due to the engagement of the distal end segment 18, 29, 39 with the inner surface of the cavity 6, the protective element 1, 2, 3 is flexed such that the protective arm 12, 22, 32 is urged away from the needle shaft.

In the activated position shown in Fig. 5b, the protective element 1, 2, 3 has been withdrawn from the housing 4 and has snapped into its natural position in which the protective arm 12, 22, 32 has moved into an oblique orientation with respect to the needle shaft, thereby safely covering the needle tip. Accordingly, the protective element 1, 2, 3 can be fully separated from the housing 4 (e.g. the cannula holder of the needle assembly) without the risk of inadvertent needle pricks.

Fig. 5c illustrates a detail view of the housing 4 as seen from the proximal side. One can see that the proximal wall 10, 20, 30 of the protective element 1, 2, 3 generally has a shape which mates the cavity 6 of the housing 4. The shape of the cavity 6 is selected such that the protective element 1, 2, 3 is held within the cavity in the ready position by way of a friction fit, wherein the friction is great enough to keep the protective element 1, 2, 3, inside the housing 4 during the initial retraction of the needle 5, but allows the protective element 1, 2, 3 to be withdrawn from the housing 4 when the engaging means 7 engages with the needle tip.

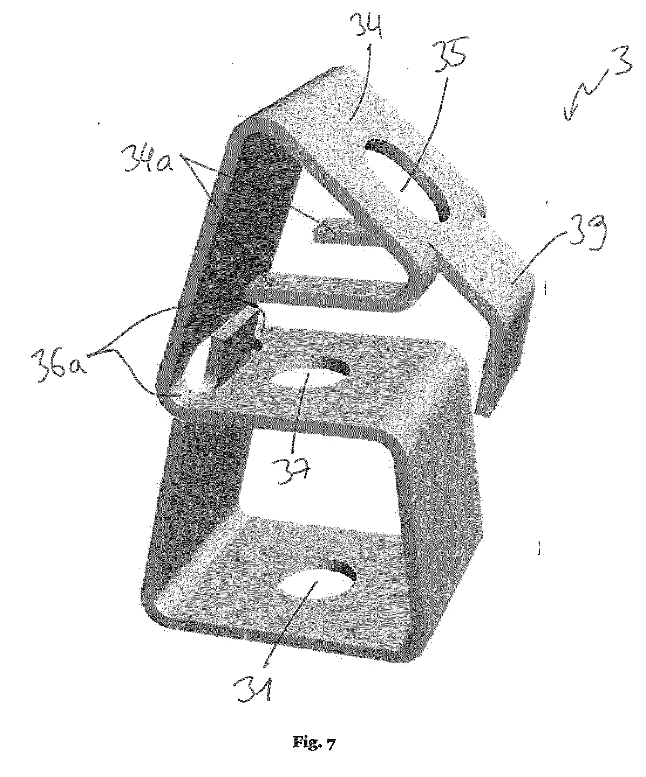

Fig. 7 illustrates a modification of the third embodiment of the generally S-shaped protective element 3 of the invention. Although the proximal side walls 33 are not shown in Fig. 7, they may also be provided in this embodiment. The same applies to the distal side walls 39a. In the depicted embodiment, the distal end segment 39 terminates in a lip which serves for maintaining the three openings 31, 35, 37 in coaxial alignment in the ready position due to the engagement of the lip with the inner cavity 6 of the housing 4. The protective element 3 in Fig. 7 also comprises two fork-shaped members 34a extending from the distal wall 34 which serve for maintaining the needle 5 in position, thereby reducing the possibility that the needle 5 escapes laterally. One can also see two joints 36a which enable to distal portion of the protective element 3 to be movable whereas the proximal portion thereof is essentially stationary, as already explained further above.

In all embodiments, the protective element 1, 2, 3 preferably consists of a unitary sheet-like body which may be made from a metal material, such as harmonic and/or stainless steel. This way, the protective element 1, 2, 3 is particularly easy to manufacture since it can be simply bent into its desired shape.

Lastly, it shall be noted that Figs. 1a-f, 2a-f and 3a-f illustrate various shapes of the proximal side walls 12, 23, 33, which may be chosen as needed and may be exchanged between the various embodiments illustrated therein. The unique aspect, however, is that in the first and second embodiment the proximal side walls 13, 23 are aligned with the respective flexible arm 12, 22 and that in the third embodiment the proximal side wall 33 is aligned with the essentially static arm 38 when in the ready position. In other words, the proximal side walls 13, 23, 33 have the same conicity as the cavity 6 in order to fit and maintain the proximal wall 10, 20, 30 in its position perpendicular to the needle axis.

## Claims

1. A protective element (1; 2; 3) for a medical needle assembly, the medical needle assembly comprising a housing (4) and a needle (5) having a needle shaft and a needle tip, the protective element (1; 2; 3) comprising:
a proximal wall (10; 20; 30) which is transversely arrangeable on the needle shaft, the proximal wall (10; 20; 30) comprising a first opening (11; 21; 31) through which the needle shaft can extend;
a protective arm (12; 22; 32) extending from the proximal wall (10; 20; 30) in a distal direction, the protective arm (12; 22; 32) being adapted for covering the needle tip in an activated position; and
two proximal side walls (13; 23; 33) extending from opposing sides of the proximal wall (10; 20; 30) in the distal direction, the two proximal side walls (13; 23; 33) being adapted for engaging with a cavity (6) of the housing (4) in a ready position.

2. The protective element (1; 2; 3) of claim 1, wherein the protective arm (12; 22; 32) comprises a distal wall (14; 24; 34) comprising a second opening (15; 25; 35) through which the needle shaft can extend in the ready position.

3. The protective element (1) of claim 1 or 2, further comprising an essentially stationary arm (16) extending from the proximal wall (10) in the distal direction, the essentially stationary arm (16) terminating in a curved lip (17).

4. The protective element (2) of claim 1 or 2, further comprising an essentially stationary arm (26) extending from the proximal wall (20) in the distal direction, the essentially stationary arm (26) comprising a transverse segment (27) comprising a third opening (28) through which the needle shaft can extend.

5. The protective element (1; 2) of any of the preceding claims, wherein the protective arm (12; 22) is flexible relative to the proximal wall (10; 20).

6. The protective element (1; 2) of any of the preceding claims, wherein the protective element (1; 2) is essentially G-shaped.

7. The protective element (3) of claim 1 or 2, wherein the protective arm (32) comprises a transverse segment (36) comprising a third opening (37) through which the needle shaft can extend.

8. The protective element (3) of the preceding claim, wherein the portion of the protective element (3) proximal from and including the transverse segment (36) is essentially stationary and wherein the portion of the protective element (3) distal from the transverse segment (36) is flexible relative to the transverse segment (36).

9. The protective element (3) of any of the preceding claims 7 or 8, further comprising an essentially stationary arm (38) extending from the proximal wall (30) in the distal direction towards the transverse segment (36) such that the transverse segment (36) is essentially stationary relative to the proximal wall (30).

10. The protective element (3) of any of the preceding claims 7-9, wherein the protective element (3) is essentially S-shaped.

11. The protective element (1; 2; 3) of any of the preceding claims, wherein the first opening (11; 21; 31), the second opening (15; 25; 35) and / or the third opening (28; 37) does not contact the needle shaft.

12. The protective element (1; 2; 3) of any of the preceding claims, wherein the protective arm (12; 22; 32) comprises a distal end segment (18; 29; 39) adapted for engaging with the cavity (6) of the housing (4) in the ready position, thereby forcing the protective arm (12; 22; 32) into a first position in which the first opening (11; 21; 31) and the second opening (15; 25; 35) are aligned relative to the needle axis.

13. The protective element (1; 2; 3) of the preceding claim, wherein the protective arm (12; 22; 32), when removed from the housing (4), is adapted for moving into a second position in which the second opening (15; 25; 35) is misaligned relative to the needle axis.

14. The protective element (1; 2; 3) of any of the preceding claims 2-13, further comprising two distal side walls (19; 29a; 39a) forming, together with the distal wall (14; 24; 34), a box-shaped distal portion of the protective element (1) adapted for collecting blood leaking from the needle tip in the activated position.

15. A medical needle assembly, comprising:
a needle (5) having a needle shaft and a needle tip;
a protective element (1; 2; 3) according to any of the preceding claims 1-14; and
a housing (4), preferably a cannula holder (4), having a cavity (6) adapted for receiving the protective element (1; 2; 3).
